# EUROPÄISCHE PATENTSCHRIFT

(11) **EP 2 641 620 B1**
(45) Veröffentlichungstag und Bekanntmachung des Hinweises auf die Patenterteilung: **10.06.2015**
(21) Anmeldenummer: 13160490.2
(22) Anmeldetag: 21.03.2013
(51) Int. Cl.: A61L 9/12

(54) **Duftmittelkartusche**
Scent agent cartridge
Cartouche d'agent odorant

(30) Priorität: 23.03.2012 DE 102012204710
(43) Veröffentlichungstag der Anmeldung: 25.09.2013
(73) Patentinhaber: MAHLE Behr GmbH & Co. KG, 70469 Stuttgart (DE)
(72) Erfinder: Stiehler, Dipl.-Ing. Daniela, 70597 Stuttgart (DE); Komowski, Dipl.-Ing. Michael, 71263 Weil der Stadt (DE); Rais, Dr. rer. nat. Thomas, 71672 Marbach/Neckar (DE)
(74) Vertreter: Grauel, Andreas

(56) Entgegenhaltungen:
- EP-A1- 2 143 577
- WO-A1-00/12143
- WO-A2-2009/003704
- DE-A1- 10 327 859

## Beschreibung

### Technisches Gebiet

Die Erfindung betrifft eine Duftmittelkartusche, insbesondere für Beduftungsvorrichtungen für Kraftfahrzeuge, nach dem Oberbegriff von Anspruch 1.

### Stand der Technik

Bei Kraftfahrzeugen sind Beduftungsvorrichtungen bekannt. Dabei wird ein Duftmittel von einem Duftmittelträger getragen in einer Kartusche angeordnet und in eine Beduftungsvorrichtung eingebracht. Die Beduftungsvorrichtung steuert durch Öffnen und Schließen der Öffnungen der Kartusche den Duftmittelaustrag, um die Luft des Innenraums eines Fahrzeuges mit einem Duftmittel zu bereichern.

Dazu werden heute Kartuschen verwendet, die den Duft in reiner Form oder auf einen Duftmittelträger fixiert aufweisen und die in die Beduftungsvorrichtung eingebracht werden, wobei mittels eines Deckels der Beduftungsvorrichtung die Duftmittelkartusche gasdicht abgeschlossen werden soll. In der DE 103 27 859 A1 oder in der EP 2085 258 B1 ist dies alleine durch den Deckel und das Gehäuse der Kartusche mittels einer Hart auf Hart Dichtfunktion realisiert, was dazu führt, dass entweder der gasdichte Abschluss nicht realisierbar ist oder eine Dichtung verwendet werden muss, die den gasdichten Abschluss realisiert.

Wird keine Dichtung verwendet, so tritt eine gewisse geringe Menge des Duftstoffs ständig aus, was zu einer ungewollten Dauerbeduftung des Fahrzeuginnenraums des Kraftfahrzeugs führt, was auf Dauer eher als störend empfunden wird. Zum einen kann eine Dauerdosierung des Duftstoffs auftreten, was als störend, weil ungewollt, empfunden wird und zum anderen kann es zu einer Duftadaption der Nase kommen, dass der Duftstoff nicht mehr wahrgenommen werden würde und zur Erzielung eines vergleichbaren Riecherlebnisses eine immer höhere Dosierung notwendig werden würde.

Bei Beduftungsvorrichtungen mit Abdichtung ist die Dichtung ein Bauteil, welches den Duftstoffen auf Dauer ausgesetzt ist. Bei den Inhaltsstoffen des Duftstoffs ist die Dichtung ständig verschiedenen Stoffen ausgesetzt, wie beispielsweise ätherischen Ölen oder Lösungsmitteln, Alkoholen, evtl. auch Säuren oder anderen Stoffen, die für die heutigen für Dichtungen geeignete Materialien, wie beispielsweise weiche Kunststoffe, Plastomere oder Elastomere, eher als materialschädigend gelten. Da bei Fahrzeugen eine Lebensdauer von mehr als zehn Jahren für die im Fahrzeug verbauten Bauteile erwartet wird, ist die Dichtung ein Bauteil, das höchsten Ansprüchen genügen müsste. Hinzu kommt, dass nach dem Gesetz von Arrhenius sich die Reaktionsgeschwindigkeit verdoppelt, wenn die Temperatur sich um 10 K erhöht. Hierbei sind die Reaktionen zwischen Duftinhaltsstoffen und den Dichtmaterial zu betrachten, die negativ sein können. Dies ist im Hinblick auf die in einem Fahrzeug auch vorherrschenden Temperaturen ein weiterer belastender Faktor für die Dichtung, weil damit die Anforderungen an die Dichtung und an das Material der Dichtung noch weiter steigen. Dies alles führt zu einem Kostenaufwand, der deutlich überhöht ist.

### Darstellung der Erfindung, Aufgabe, Lösung, Vorteile

Es ist die Aufgabe der Erfindung, eine Duftmittelkartusche zu schaffen, die gegenüber dem Stand der Technik verbessert ist und einfach herstellbar ist und dafür sorgt, dass im Betrieb einer Beduftungsvorrichtung eine dauerhaft gute Abdichtung des Duftstoffs gewährleistet werden kann.

Dies wird mit den Merkmalen von Anspruch 1 erreicht.

Ein Ausfuhrungsbeispiel sieht eine Duftmittelkartusche mit einem Gehäuse vor, Welches einen Aufnahmeraum umschließt zur Aufnahme eines Duftmittelträgers mit einem Duftmittel, wobei das Gehäuse einen Wandbereich mit zumindest einer Öffnung aufweist zum Austreten des Duftmittels durch die Öffnung und das Gehäuse einen Wandbereich aufweist zum Öffnen und Einbringen des Duftmittelträgers, wobei der Wandbereich zum Austreten des Duftmittels von einer Dichtung umfasst ist zur dichtenden Anlage eines Abschlussdeckels. Dies hat den Vorteil, dass die Dichtung mit der Kartusche getauscht wird und daher die Anforderungen an die Dichtung hinsichtlich der Dauerhaltbarkeit deutlich reduziert sind.

Dabei ist es vorteilhaft, wenn der Wandbereich mit der zumindest einen Öffnung zum Austreten des Duftmittels von dem Wandbereich zum Öffnen und Einbringen des Duftmittelträgers getrennt ist.

Auch ist es zweckmäßig, wenn der Wandbereich mit der zumindest einen Öffnung zum Austreten des Duftmittels und der Wandbereich zum Öffnen und Einbringen des Duftmiftelträgers ein Bauteil ist. Dadurch ist es vorteilhaft, wenn der Wandbereich mit der Dichtung um die Öffnung oder um die Öffnungen herum als wechselbares Teil ausgebildet ist, weil dadurch dieses Bauteil getrennt vom restlichen Gehäuse hergestellt werden kann. So kann beispielswiese die Wandung als Deckel in einem anderen Verfahren hergestellt werden als das restliche Gehäuse. Das restliche Gehäuse kann beispielsweise aus einem harten Kunststoff als Spritzgussteil hergestellt werden, wobei das entsprechende Deckelteil beispielsweise in einem Zwekomponentenspritzgussverfahren hergestellt werden kann.

Auch ist es zweckmäßig, wenn die Dichtung an dem Wandbereich mit der zumindest einen Öffnung angebracht ist und die zumindest eine Öffnung in einer Ebene umgibt.

Zweckmäßig ist auch, wenn der Wandbereich mit der zumindest einen Öffnung als Wandbereich zum Öffnen und Einbringen des Duftmittelträgers vom Gehäuse abnehmbar ist und einen Deckel des Gehäuses bildet.

Dabei ist es auch vorteilhaft, wenn die zumindest eine Öffnung durch zumindest einen Trennsteg oder Trennstege in eine Mehrzahl von zusammenhängenden oder getrennten Öffnungen unterteilt ist. So kann mittels eines kreuzförmigen Trennstegs eine Anordnung von vier Öffnungen erzeugt werden.

Weiterhin ist es vorteilhaft, wenn die Dichtung mit dem Wandbereich verbunden ist, wie insbesondere an den Wandbereich angespritzt oder in eine Aufnahme für die Dichtung im Wandbereich eingelegt.

Auch ist es zweckmäßig, wenn der Deckel und die Dichtung miteinander als zwei Komponenten Spritzgussteil hergestellt werden, wobei der Deckel als Hartkomponente und die Dichtung als Weichkomponente gespritzt wird.

Weiterhin ist es zweckmäßig, wenn der Deckel und die Dichtung miteinander verbunden hergestellt sind, wobei der Deckel als Hartkomponente ausgeführt ist und die Dichtung als Weichkomponente ausgeführt und als Einlegeteil darin eingelegt ist.

Auch ist es vorteilhaft, wenn der Wandbereich zum Öffnen und Einbringen des Duftmittelträgers mit dem Gehäuse über ein Scharnier, vorzugsweise über ein Filmscharnier verbunden ist.

Weitere vorteilhafte Ausgestaltungen sind durch die nachfolgende Figurenbeschreibung und durch die Unteransprüche beschrieben.

### Kurze Beschreibung der Zeichnungen

Nachstehend wird die Erfindung auf der Grundlage zumindest eines Ausführungsbeispiels anhand der Zeichnungen näher erläutert. Es zeigen:
- Fig. 1a: eine schematische perspektivische Ansicht einer Duftmittelkartusche mit einem Abschlussdeckel zum Verschließen der Kartusche,
- Fig. 1b: eine schematische perspektivische Ansicht einer Duftmittelkartusche mit einem Abschlussdeckel zum Verschließen der Kartusche im Halbschnitt,
- Fig. 1c: eine schematische Ansicht einer Duftmittelkartusche mit einem Abschlussdeckel zum Verschließen der Kartusche im Schnitt,
- Fig. 2a: eine schematische perspektivische Ansicht einer mit einem Abschlussdeckel verschlossenen Duftmittelkartusche,
- Fig. 2b: eine schematische perspektivische Ansicht einer mit einem Abschlussdeckel verschlossenen Duftmittelkartusche im Halbschnitt,
- Fig. 2c: eine schematische Ansicht einer mit einem Abschlussdeckel verschlossenen Duftmittelkartusche im Schnitt,
- Fig. 3: ein Ausführungsbeispiel einer Duftmittelkartusche,
- Fig. 4: eine Anordnung eines Duftmittelträgers in einem Gehäuse,
- Fig. 5: eine Darstellung einer Duftmittelkartusche im Schnitt, Fig. 6 ein Ausführungsbeispiel einer Duftmitelkartusche,
- Fig. 7: eine Darstellung einer Duftmittelkartusche im Halbschhitt,
- Fig. 8: ein Ausführungsbeispiel einer Duftmittelkartusche,
- Fig. 9: eine Darstellung einer Duftmittelkartusche in perspektivischer Ansicht,
- Fig. 10a: eine schematische perspektivische Ansicht einer Duftmittelkartusche,
- Fig. 10b: eine schematische perspektivische Ansicht einer Duftmittelkartusche,
- Fig. 10c: eine schematische perspektivische Ansicht einer Duftmittelkartusche,
- Fig. 11a: eine Dichtung im Schnitt,
- Fig. 11b: eine Dichtung im Schnitt,
- Fig. 11c: eine Dichtung im Schnitt,
- Fig. 12: eine schematische perspektivische Ansicht einer Duftmittelkartusche,
- Fig. 13: eine schematische perspektivische Ansicht einer Duftmittelkartusche mit Dichtung, und
- Fig. 14: eine schematische perspektivische Ansicht einer Duftmittelkartusche.

### Bevorzugte Ausführung der Erfindung

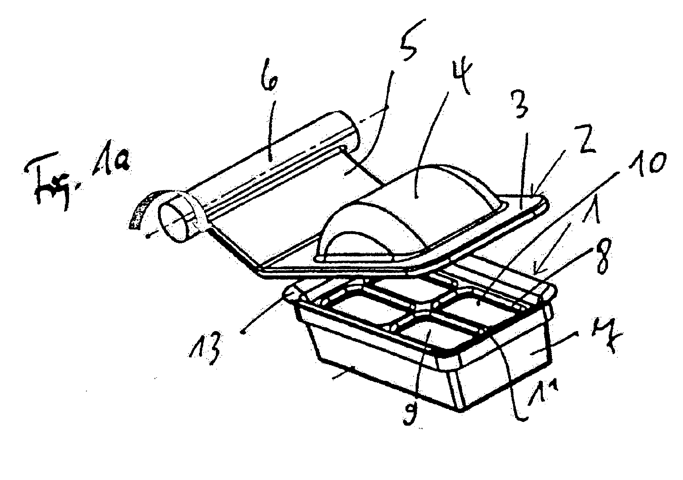

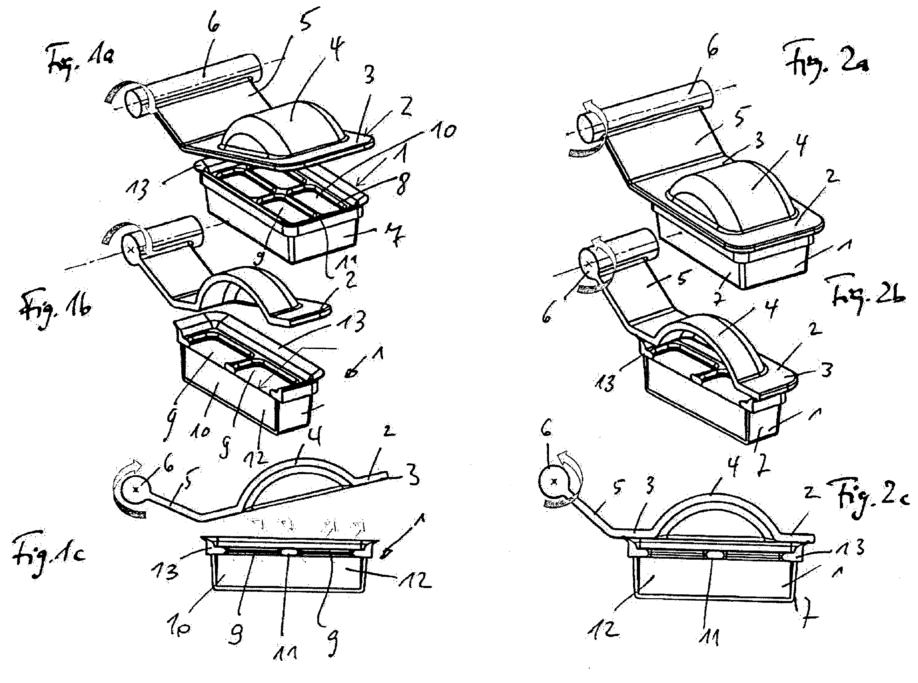

Die Figuren 1a bis 1c und die Figuren 2a bis 2c zeigen eine Anordnung einer Duftmittelkartusche 1 relativ zu einem Abschlussdeckel 2 der in einer Beduftungsvorrichtung gesteuert die Duftmittelkartusche 1 öffnen oder verschließen kann. In den Figuren 1a bis 1c ist die Duftmittelkartusche 1 durch den Abschlussdeckel 2 geöffnet und in den Figuren 2a bis 2c ist die Duftmittelkartusche 1 durch den Abschlussdeckel 2 verschlossen. Im geöffneten-Zustand

Der Abschlussdeckel 2 weist eine Platte 3 auf, die einen ausgewölbten Bereich 4 aufweist, wobei die Platte 3 über einen Verbindungsbereich 5 mit einer drehbaren Welle 6 verbunden ist, so dass der Abschlussdeckel 2 durch die Verdrehung der Welle 6 entweder auf die Duftmittelkartusche 1 aufgesetzt werden kann, wie es in den Figuren 2a bis 2c gezeigt ist oder von der Duftmittelkartusche 1 abgehobeh werden kann, so dass ein Duftmittel aus der Duftmittelkartusche 1 entweichen kann.

Die Duftmittelkartusche 1 weist dazu ein Gehäuse 7 auf, das nach fünf Seiten geschlossen oder im Wesentlichen quaderförmig ausgebildet ist, wobei an der Oberseite des Gehäuses Öffnungen 9 vorgesehen sind, durch welche ein Duftmittel aus der Duftmittelkartusche 1 austreten kann. Zur Halterung eines Duftmittelträgers 10, welcher im Inneren des Gehäuses 7 der Duftmittelkartusche 1 angeordnet werden kann, weist das Gehäuse 7 einen Wandbereich 8 mit Stegen 11 auf, die dazu dienen, den Duftmittelträger 10 im Aufnahmeraum 12 zu halten. Um die Öffnungen 9 herum weist der Wandbereich 8 im Wesentlichen in der Ebene des Wandbereichs 8 eine Dichtung 13 auf, die der Abdichtung der Duftmittelkartusche dient, so dass die Platte 3 des Abschlussdeckels 2 sich an der Dichtung 13 abdichtend anlegen kann, um die Duftmittelkartusche 1 zu verschließen und abzudichten.

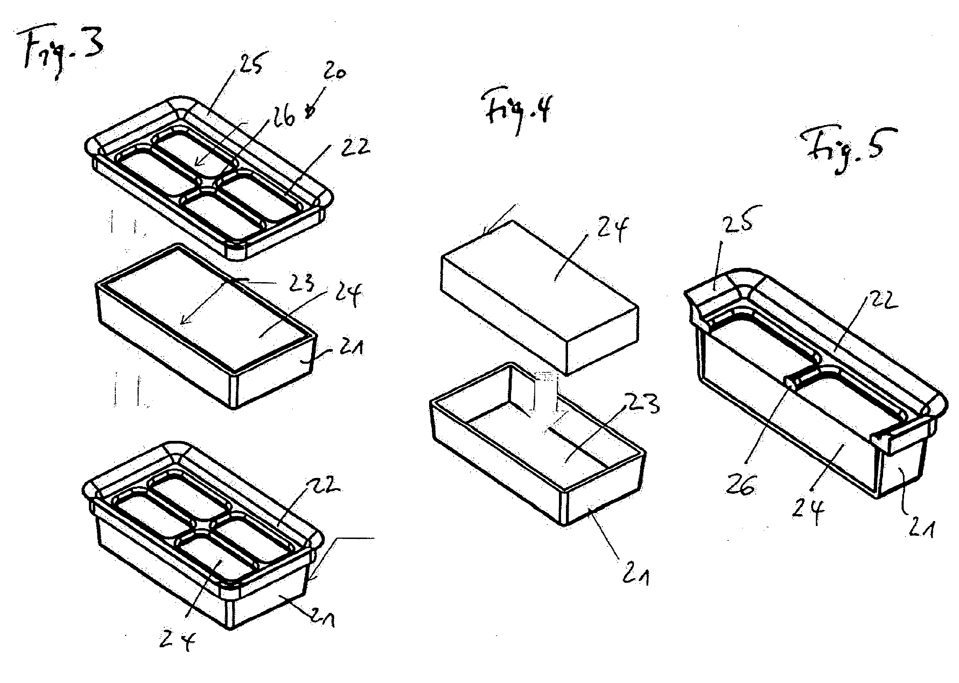

Die Figur 3 zeigt in einem ersten Ausführungsbeispiel eine Duftmittelkartusche 20 mit einem Gehäuse 21, das quaderförmig ausgebildet ist und im Wesentlichen fünf Seiten eines Quaders mit Wänden abschließt. Im Innenraum des Gehäuses 21 ist ein Aufnahmeraum 23 zu Anordnung eines Duftmittelträgers 24 vorgesehen. Der Deckel 22 kann auf das Gehäuse aufgesetzt werden, so dass der Deckel das Gehäuse 21 nach oben abschließt. Der Deckel 22 ist dabei als einteiliges Element als Wandbereich ausgebildet, das aus einem elastischen weichen kunstotoffmaterial ausgebildet ist. Der Deckel ist vorzugsweise in einem Einkomponenten-Spritzgussverfahren herstellbar. Dabei ist sowohl die Deckelstruktur 22 als auch die umlaufende Dichtlippe 25 aus einem Material hergestellt, wie gespritzt.

Die Figur 3 zeigt unten den Zusammenbau des Gehäuses 21 mit dem Deckel 22, wobei der Duftmittelträger 24 im Innenraum in einem Aufnahmeraum des Gehäuses 21 angeordnet ist.

Die Figur 4 zeigt wie der Duftmittelträger 24, hier vorteilhaft als quaderförmiges Element, in den Aufnahmeraum 23 des Gehäuses 21 einsetzbar ist.

Die Figur 5 zeigt einen Schnitt durch eine Duftmittelkartusche, die ein Gehäuse 21 mit einem Duftmittelträger 24 aufweist, wobei der Deckel 22 sowohl ein kreuzförmiges Halteelement 26 als Trennstege aufweist, als auch eine umlaufende Dichtlippe 25.

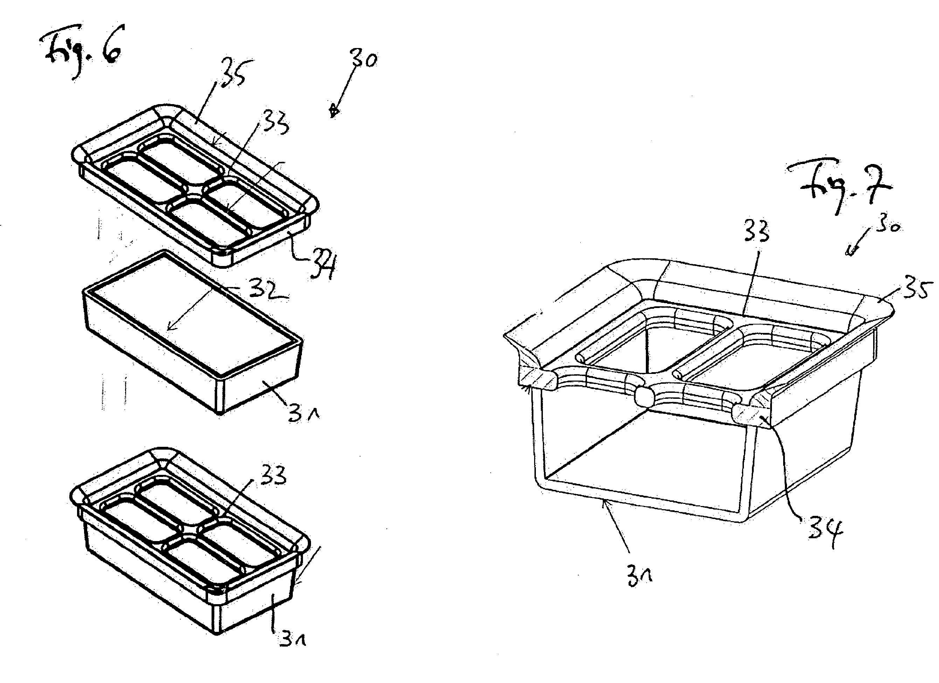

Die Figuren 6 und 7 zeigen ein weiteres Ausführungsbeispiel der Erfindung, bei dem die Duftmittelkartusche 30 ein Gehäuse 31 mit einem darin angeordneten Duftmittelträger 32 aufweist, wobei der Deckel 33 aus einem harten Bodenteil 34 gebildet ist, an dem eine elastische Dichtlippe 35 angeordnet ist. Unten in der Figur 6 ist der Zusammenbau des Gehäuses 31 mit dem Deckel 33 dargestellt.

Die Figur 7 zeigt die Anordnung des Gehäuses 31 mit dem Deckel 33 mit der Dichtung 35 noch einmal in einem Schnitt. Dabei ist zu erkennen, dass der Deckel aus einem harten Bodenbereich 34 und der elastischen weichen Dichtlippe gebildet ist, wobei das Bodenteil in einem Spritzgussprozess mit der Dichtlippe in einen Zweikomponenten-Spritzgussverfahren hergestellt ist, so dass der Bodenbereich 34 des Deckels aus einem harten Kunststoffmaterial gebildet werden kann, wobei die Dichtlippe 35 aus einem weichen, elastischen Kunststoff anspritzbar ist.

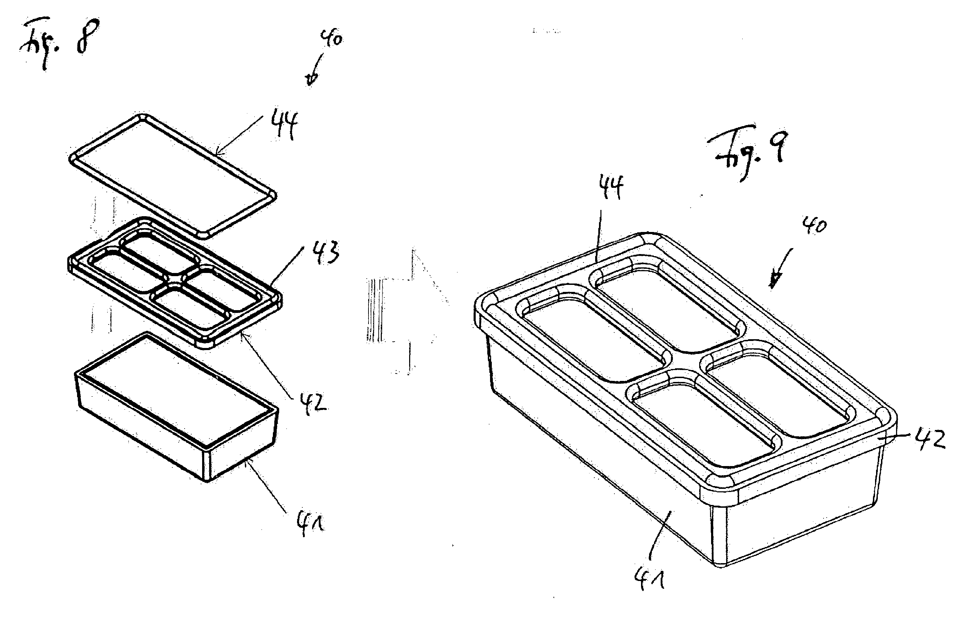

Die Figuren 8 und 9 zeigen ein weiteres Ausführungsbeispiel der Erfindung, bei dem eine Duftmittelkartusche 40 gezeigt ist, die ein Gehäuse 41 und einen Deckel 42 aufweist, wobei das Gehäuse und auch der Deckel aus einem harten Kunststoffmaterial herstellbar ist, wobei der Deckel eine umlaufende Nut 43 aufweist, in die eine Dichtung 44, wie eine Rundschnurdichtung oder ein Dichtungsrahmen oder ähnliches einlegbar ist.

Die Figur 9 zeigt die Anordnung der Kartusche 40 mit dem Gehäuse 41 und dem Deckel 42, wobei die Dichtung 44 in die umlaufende Nut eingelegt ist.

Bei den Ausführungsbeispielen der Figuren 1a bis 9 ist jeweils der Deckel als Wandbereich des Gehäuses mit der zumindest einen Öffnung oder mit den Öffnungen versehen, durch welche das Duftmittel aus der Kartusche austreten kann, wenn der Abschlussdeckel von der Dichtung abgehoben ist. Dabei ist der Deckel des Gehäuses als ein abnehmbarer Wandbereich des Gehäuses ausgebildet.

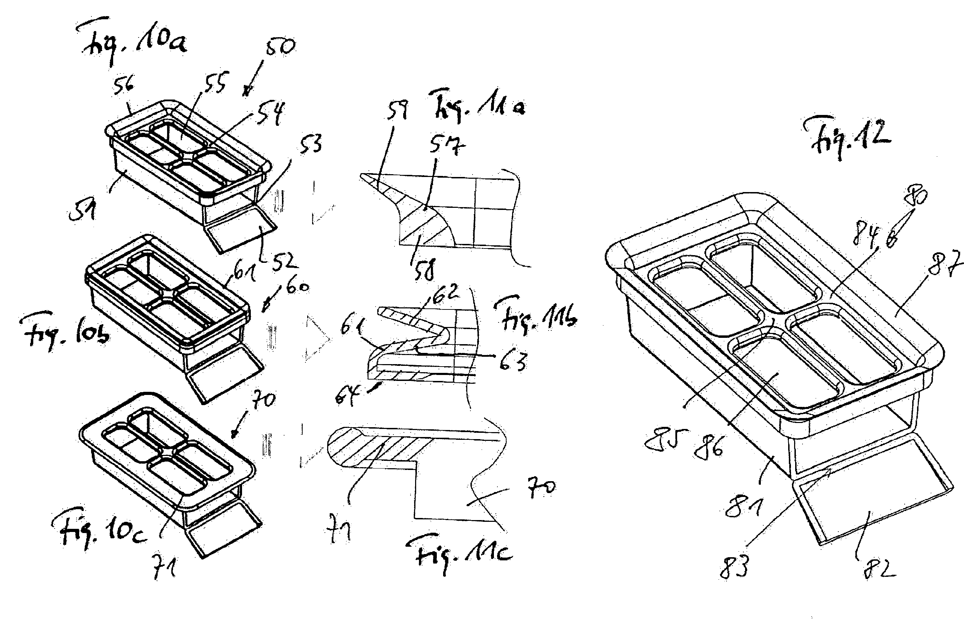

In den Figuren 10a bis 14 ist ein alternatives Ausführungsbeispiel dargestellt, bei welchem der Deckel mit den Öffnungen ein mit dem Gehäuse fest verbundener Wandbereich darstellt, wobei die Öffnung zum Einführen des Duftmittelträgers in einer anderen Wand des Gehäuses eingebracht ist, wobei diese Wand vorteilhaft zu öffnen und wieder zu verschließen ist.

Die Figuren 10a bis 10c zeigen eine Duftmittelkartusche 50 mit einem Gehäuse 51, wobei eine Gehäusewandung 52 zu öffnen und zu schließen ist, wobei die Wandung vorteilhaft über ein Scharnier 53 auf und zu klappbar ist. Die Oberseite des Gehäuses ist als feststehende mit dem Gehäuse verbundene Wandung 54 ausgebildet, die Öffnungen 55 aufweist, wobei die Wandung mit einer umlaufenden Dichtung 56 versehen ist. Im Ausführungsbeispiel der Figur 10a ist eine Dichtung angeordnet, wie angespritzt, die in der Figur 11a mit den Bezugszeichen 57 versehen ist. Dort ist zu erkennen, dass die Dichtung einen breiten Fuß 58 aufweist, der im Winkel von etwa 45° nach oben und nach außen sich spitz verjüngt, so dass er in einer Spitze 59 resultiert, die bei Auflage eines Abschlussdeckels sich an diesen abdichtend anlegt.

Die Figur 10b zeigt eine Duftmittelkartusche 60 ähnlich der Duftmittelkartusche 50 der Figur 10a, wobei die Dichtung 61 gemäß Figur 11b als Dichtung ausgebildet ist, die zick-zack-förmig ausgebildet ist, wobei ein oberer Schenkel 62 mit einem mittleren Schenkel 63 verbunden ist, der mit einem unteren Schenkel 64 wiederum verbunden ist. Die Dichtung ist aus elastomerem oder weichem Kunststoffmaterial hergestellt und wird bei Beaufschlagung durch einen Abschlussdeckel ziehharmonikaartig zusammengefaltet.

Die Figur 10c zeigt eine Duftmittelkartusche 70 mit einer Dichtung 71, wobei die Dichtung 71 in der Figur 11c näher gezeigt ist. Sie ist in einer Ebene der Wandung lateral abstehend ausgebildet, wobei am seitlichen Ende die Dichtung einen etwa kreisförmig verdickten Bereich aufweist, an den der Abschlussdeckel sich anlegen kann um die Duftmittelkartusche abzudichten.

Die Figur 12 zeigt eine Duftmittelkartusche 80 mit einem Gehäuse 81, wobei eine Gehäusewandung 82 seitlich wegklappbar ist, wobei ein Scharnier 83, wie vorteilhaft ein Filmscharnier, zwischen dem Gehäuse und der Gehäusewandung 82 vorhanden ist, um die Gehäusewandung 82 klappbar zu gestalten. Das Gehäuse ist oben mit einem Deckel 84 versehen, das einen kreuzförmigen Trennsteg 85 aufweist zur Halterung des in dem Gehäuse anzuordnenden Duftmittelträgers. Umlaufend um die Gehäusewandung mit den Öffnungen 86 ist eine Dichtung 87 ausgebildet, die zur Abdichtung eines Abschlussdeckels dient, der in einer Beduftungsvorrichtung vorteilhaft gesteuert auf die Duftmittelkartusche aufgesetzt oder von dieser abgehoben werden kann.

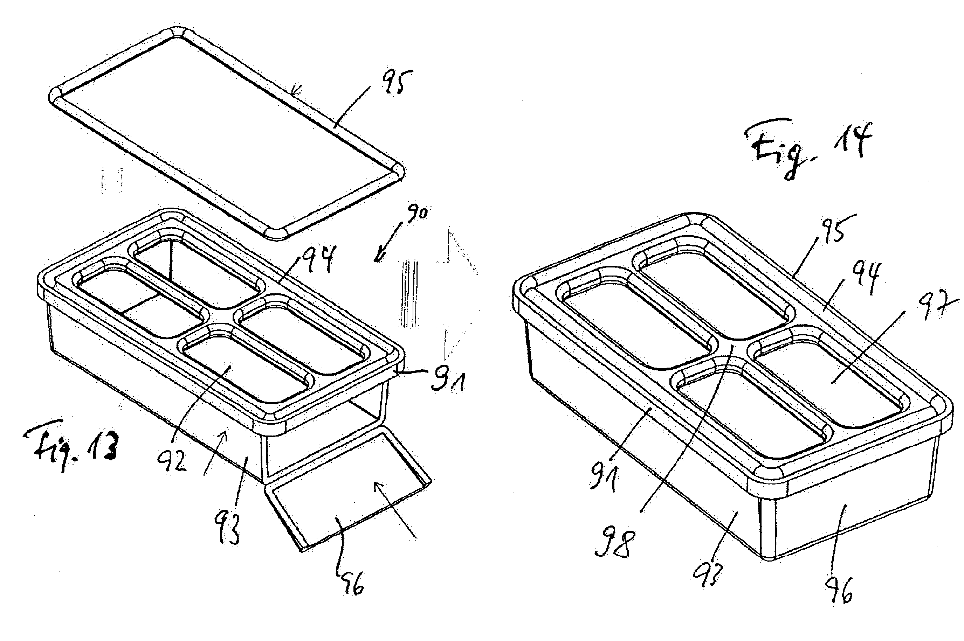

Die Figur 13 zeigt ein Ausführungsbeispiel einer erfindungsgemäßen Duftmittelkartusche 90 bei der die Wandung 91 mit den Öffnungen 92 zum entweichen des Duftmittels als feste mit dem Gehäuse 93 verbundene Wandung ausgebildet ist, wobei die Wandung außen eine umlaufende Nut 94 aufweist, in die eine Dichtung 95 aufnehmbar angeordnet werden kann. Dabei zeigt die Figur 14 die Anordnung der Dichtung 95 in der Nut 94 der Wandung 91 des Gehäuses 93. Dabei ist auch der seitliche Wandungsbereich 96 zum Einführen eines Duftmittelträgers 97 zugeklappt, wobei der Duftmittelträger 97 durch den kreuzförmigen Steg 98 in der Kartusche gehalten wird.

Die Figuren 1a bis 12 zeigen Ausführungsbeispiele, bei welchen eine Dichtung vorteilhaft an einen Deckel oder an das Gehäuse angespritzt ist, wobei verschiedene Ausführungsbeispiele dargestellt sind, so dass zumindest die eine Gehäusewandung entweder als hartes Kunststoffteil oder als weiches Kunststoffteil gespritzt werden kann, wobei die Dichtung bei dem harten Kunststoffteil als weiches Kunststoffteil in einem Zweikomponentenverfahren angespritzt werden kann oder bei Ausbildung des Wandbereichs als Deckel als weiches Kunststoffteil mit dem Deckel gemeinsam gespritzt wird. In den Ausführungsbeispielen der Figuren 13 und 14 ist die Dichtung eine elastische Dichtung die als Dichtungsrahmen oder als Dichtungsschnur eingelegt wird, wobei der Wandungsbereich 91 bevorzugt als hartes Kunststoffteil gebildet ist, wie beispielsweise auch das Gehäuse 93 der Figuren 13 und 14.

Die erfindungsgemäßen Duftmittelkartuschen sind so ausgebildet, dass die Dichtung an der Kartusche selbst angeordnet ist, so dass beim Austausch der Duftmittelkartusche nach Verbrauch des Duftmittels und beim Einsetzen einer neuen gefüllten Duftmittelkartusche auch die Dichtung mit ausgetauscht wird, was dazu führt, dass die mittlere Lebensdauer der Dichtung deutlich kürzer ist als die Lebensdauer der Beduftungsvorrichtung, so dass die Verwendung von Elastomeren oder weichen Kunststoffen (Plastomere) als Dichtung im Wesentlichen gut möglich ist, obwohl die Inhaltsstoffe der Duftmittel gegebenenfalls auch nachteilige Eigenschaften für die Dichtung aufweisen können. Durch den Austausch der Kartusche mit der Dichtung stellt die Lebensdauer keinerlei Probleme dar, für die Dauerhaltbarkeit der Dichtung über diesen eher kurzen Zeitraum.

## Patentansprüche

1. Duftmittelkartusche mit einem Gehäuse, welches einen Aufnahmeraum umschließt zur Aufnahme eines Duftmittelträgers mit einem Duftmittel, wobei das Gehäuse einen Wandbereich mit zumindest einer Öffnung aufweist zum Austreten des Duftmittels durch die Öffnung und das Gehäuse einen Wandbereich aufweist zum Öffnen und Einbringen des Duftmittelträgers, wobei der Wandbereich zum Austreten des Duftmittels von einer Dichtung umfasst ist zur dichtenden Anlage eines Abschlussdeckels, wobei die Dichtung an dem Wandbereich mit der zumindest einen Öffnung angebracht ist und die zumindest eine Öffnung in einer Ebene umgibt, wobei die Dichtung mit dem Wandbereich verbunden ist, in dem sie an den Wandbereich angespritzt ist, wobei der Deckel und die Dichtung miteinander als zwei Komponenten Spritzgussteil hergestellt ist, wobei der Deckel als Hartkomponente und die Dichtung als Weichkomponente gespritzt ist.

2. Duftmittelkartusche nach Anspruch 1, **dadurch gekennzeichnet, dass** der Wandbereich mit der zumindest einen Öffnung zum Austreten des Duftmittels von dem Wandbereich zum Öffnen und Einbringen des Duftmittelträgers getrennt ist.

3. Duftmittelkartusche nach Anspruch 1, **dadurch gekennzeichnet, dass** der Wandbereich mit der zumindest einen Öffnung zum Austreten des Duftmittels und der Wandbereich zum Öffnen und Einbringen des Duftmittelträgers ein Bauteil ist.

4. Duftmittelkartusche nach einem der vorhergehenden Ansprüche, **dadurch gekennzeichnet, dass** der Wandbereich mit der zumindest einen Öffnung als Wandbereich zum Öffnen und Einbringen des Duftmittelträgers vom Gehäuse abnehmbar ist und einen Deckel des Gehäuses bildet.

5. Duftmittelkartusche nach einem der vorhergehenden Ansprüche, **dadurch gekennzeichnet, dass** die zumindest eine Öffnung durch Trennstege in eine Mehrzahl von zusammenhängenden oder getrennten Öffnungen unterteilt ist.

6. Duftmittelkartusche nach einem der vorhergehenden Ansprüche, **dadurch gekennzeichnet, dass** der Wandbereich zum Öffnen und Einbringen des Duftmittelträgers mit dem Gehäuse über ein Scharnier, vorzugsweise über ein Filmscharnier verbunden ist.

## Claims

1. Fragrance cartridge having a housing which encloses an accommodating chamber for accommodating a fragrance carrier with a fragrance, wherein the housing has a wall region with at least one opening, for the fragrance to be discharged through the opening, and the housing has a wall region for opening purposes and for introduction of the fragrance carrier, wherein the wall region for discharging the fragrance is encompassed by a seal, for the sealing abutment of a closing-off cover, wherein the seal is fitted on the wall region with the at least one opening and surrounds the at least one opening in a single plane, wherein the seal is connected to the wall region by being injection moulded thereon, wherein the cover and the seal are produced together as a two-component injection moulding, wherein the cover is injection moulded in the form of a hard component and the seal is injection moulded in the form of a soft component.

2. Fragrance cartridge according to claim 1, **characterized in that** the wall region with the at least one opening for discharging the fragrance is separate from the wall region for opening purposes and for introduction of the fragrance carrier.

3. Fragrance cartridge according to claim 1, **characterized in that** the wall region with the at least one opening for discharging the fragrance and the wall region for opening purposes and introduction of the fragrance carrier form a single component.

4. Fragrance cartridge according to one of the preceding claims, **characterized in that** the wall region with the at least one opening, as the wall region for opening purposes and introduction of the fragrance carrier, is removable from the housing and forms a cover of the housing.

5. Fragrance cartridge according to one of the preceding claims, **characterized in that** the at least one opening is subdivided, by separating crosspieces, into a plurality of contiguous or separate openings.

6. Fragrance cartridge according to one of the preceding claims, **characterized in that** the wall region for opening purposes and introduction of the fragrance carrier is connected to the housing via a hinge, preferably via a film hinge.

## Revendications

1. Cartouche de produit parfumé comprenant un boîtier qui enferme un espace récepteur pour recevoir un support pour produit parfumé comprenant un produit parfumé, où le boîtier présente une zone de paroi comportant au moins une ouverture servant à diffuser, à travers ladite ouverture, le produit parfumé, et le boîtier présente une zone de paroi servant à ouvrir et à introduire le support pour produit parfumé, où la zone de paroi servant à diffuser le produit parfumé est entourée par un joint d'étanchéité permettant à un couvercle de fermeture d'être en appui de façon étanche, où le joint d'étanchéité est fixé sur la zone de paroi comportant l'ouverture au moins au nombre de un et entoure dans un plan l'ouverture au moins au nombre de un, où le joint d'étanchéité est assemblé avec la zone de paroi dans laquelle il est moulé par injection sur la zone de paroi, où le couvercle et le joint d'étanchéité sont fabriqués l'un avec l'autre comme une pièce moulée par injection à deux composants, où le couvercle est injecté comme un composant dur et le joint d'étanchéité comme un composant souple.

2. Cartouche de produit parfumé selon la revendication 1, **caractérisée en ce que** la zone de paroi comportant l'ouverture au moins au nombre de un servant à diffuser le produit parfumé est séparée de la zone de paroi servant à ouvrir et à introduire le support pour produit parfumé.

3. Cartouche de produit parfumé selon la revendication 1, **caractérisée en ce que** la zone de paroi comportant l'ouverture au moins au nombre de un servant à diffuser le produit parfumé est un composant, et la zone de paroi servant à ouvrir et à introduire le support pour produit parfumé est un composant.

4. Cartouche de produit parfumé selon l'une quelconque des revendications précédentes, **caractérisée en ce que** la zone de paroi comportant l'ouverture au moins au nombre de un, en tant que zone de paroi servant à ouvrir et à introduire le support pour produit parfumé, est séparable du boîtier et forme un couvercle du boîtier.

5. Cartouche de produit parfumé selon l'une quelconque des revendications précédentes, **caractérisée en ce que** l'ouverture au moins au nombre de un est subdivisée par des barrettes de séparation, en une pluralité d'ouvertures qui communiquent ou sont séparées.

6. Cartouche de produit parfumé selon l'une quelconque des revendications précédentes, **caractérisée en ce que** la zone de paroi servant à ouvrir et à introduire le support pour produit parfumé est reliée au boîtier par une charnière, de préférence par une charnière pelliculaire.
